Europäisches Patentamt

European Patent Office

Office européen des brevets

(19)

(11) **EP 0 609 144 B1**

(12)

# FASCICULE DE BREVET EUROPEEN

(45) Date de publication et mention
de la délivrance du brevet:
**19.05.1999   Bulletin 1999/20**

(51) Int Cl.⁶: **G01N 33/543**, G01N 33/547,
G01N 33/577, G01N 33/58

(21) Numéro de dépôt: **94400166.8**

(22) Date de dépôt: **26.01.1994**

(54) **Dosage immunométrique d'un antigène ou d'un haptène**

Immunologische Bestimmung eines Antigens oder eines Haptens

Immunoassay of an antigen or a hapten

(84) Etats contractants désignés:
**BE CH DE ES GB IT LI NL SE**

(30) Priorité: **28.01.1993   FR 9300869**

(43) Date de publication de la demande:
**03.08.1994   Bulletin 1994/31**

(73) Titulaire: **COMMISSARIAT A L'ENERGIE
ATOMIQUE
75015 Paris Cédex 15 (FR)**

(72) Inventeur: **Pradelles, Philippe
F-91140 Villebon (FR)**

(74) Mandataire: **Des Termes, Monique
Société Brevatome
25, rue de Ponthieu
75008 Paris (FR)**

(56) Documents cités:
**EP-A- 0 080 109          EP-A- 0 467 078
WO-A-91/16377**

## Description

La présente invention a pour objet un procédé de dosage immunométrique d'une substance constituée par un antigène ou un haptène.

Elle s'applique particulièrement au dosage des haptènes, c'est-à-dire de petites molécules qui très souvent ne possèdent en elles-mêmes qu'un seul site de fixation d'anticorps (haptènes monoépitopiques). En effet, un haptène est une petite molécule qui ne peut pas par elle-même stimuler la synthèse d'anticorps mais qui peut induire la formation d'anticorps lorsqu'on la couple avec une protéine qui sert de porteur antigénique.

Dans le cas des haptènes dont la taille n'est pas suffisante pour être liés simultanément à 2 anticorps, on réalise donc habituellement les dosages immunologiques par compétition entre l'haptène à doser et le même haptène marqué, pour des sites anticorps en quantité limitée, fixés sur une phase solide, éventuellement par l'intermédiaire d'un autre anticorps, comme il est décrit par Pradelles et al dans Anal. Chem. 57, 1985, p. 1170, et par Renzi et al dans Trends in Cluster Headache, F. Sicuteri et al, Editors, Elsevier, NY, 1987, 125-134, dans le cas de la substance P.

D'autres dosages immunologiques tels que les dosages immunométriques du type "sandwich" ou à deux sites, dans lesquels l'antigène est fixé sur une phase solide par l'intermédiaire d'un premier anticorps, puis révélé par un second anticorps marqué et mis en excès, permettent l'obtention d'une beaucoup plus grande sensibilité, mais ils ne sont malheureusement pas applicables aux haptènes monoépitopiques.

La présente invention a précisément pour objet un procédé de dosage immunométrique, utilisable notamment pour le dosage de ces haptènes, qui permet d'obtenir une bien meilleure sensibilité que les dosages par compétition.

Selon l'invention, le procédé de dosage immunométrique d'une substance constituée par un antigène ou un haptène, comprend les étapes suivantes :

1°) mettre en contact un échantillon contenant la substance à doser avec une phase solide sur laquelle sont fixés une substance de saturation et un anticorps de capture, dit premier anticorps, spécifique d'un épitope de la substance à doser, pour lier immunologiquement la substance à doser avec l'anticorps fixé,

2°) soumettre la phase solide sur laquelle sont fixés la substance de saturation, l'anticorps et la substance à doser à un traitement au moyen d'au moins un réactif pour immobiliser la substance à doser sur la phase solide par formation de liaisons covalentes et pour dénaturer la liaison immunologique obtenue précédemment entre la substance à doser et le premier anticorps afin de rendre accessible un épitope de la substance à doser en vue d'une nouvelle réaction immunologique,

3°) mettre en contact la phase solide ainsi traitée avec un anticorps marqué, dit second anticorps, spécifique de la substance à doser,

4°) mesurer la quantité du second anticorps fixé sur la phase solide, et

5°) déterminer sur une courbe d'étalonnage la quantité de substance à doser présente dans l'échantillon à partir de la quantité du second anticorps mesurée dans la quatrième étape.

Dans ce procédé, le traitement effectué dans la deuxième étape est très important puisqu'il conduit à une immobilisation complète de la substance à doser sur la phase solide par des liaisons chimiques covalentes et à une restitution des propriétés immunologiques de la substance à doser pour qu'elle puisse à nouveau réagir immunologiquement avec un anticorps.

Généralement, ce traitement comprend

- un premier stade dans lequel on fait réagir la substance à doser liée immunologiquement au premier anticorps avec un réactif au moins bifonctionnel capable de former des liaisons covalentes, d'une part, avec la substance à doser, et, d'autre part, avec la phase solide revêtue du premier anticorps et de la substance de saturation pour immobiliser la substance à doser sur la phase solide au moyen de ce réactif, et
- un second stade dans lequel on soumet la substance à doser immobilisée sur la phase solide à un traitement de dénaturation de sa liaison immunologique avec le premier anticorps.

Le réactif bifonctionnel utilisé dans le premier stade est un réactif comportant un premier groupement fonctionnel capable de réagir chimiquement avec la phase solide, la substance de saturation et/ou le premier anticorps fixé sur la phase solide, et un second groupement fonctionnel, identique ou différent du premier, capable de réagir avec la substance à doser. Ces groupements fonctionnels peuvent être par exemple des groupements amines, des groupements acides, des groupements aldéhydes, des groupements thyrosyles, des groupements histidyles ou des groupements thiols.

Lorsque ces groupements fonctionnels sont identiques, on a un réactif homobifonctionnel ou homopolyfonctionnel.

A titre d'exemple de tels réactifs, on peut citer le glutaraldéhyde, le difluorodinitrobenzène, le disuccinimidyl subérate, le bis(maléimido)hexane, et le bis[β-(4-azido-salicylamido)éthyl] disulfure.

Lorsque les groupements fonctionnels sont différents, on a un réactif hétérobifonctionnel ou hétéropolyfonctionnel.

A titre d'exemple de tels réactifs, on peut citer le N-succinimidyl-3-(2-pyridyldithio)propionate et le succinimidyl-4-(N-maléimidométhyl)cyclohexane-1-carboxylate.

Le choix du réactif utilisé dépend non seulement de la substance à doser, mais également de la phase solide et de l'anticorps utilisés.

La phase solide peut être constituée par les phases solides généralement utilisées dans les dosages immunologiques, par exemple par des plaques de microtitration, des tubes, des membranes en matière plastique, par exemple en polystyrène, en nitrocellulose ou en polyester, des billes de verre, ou par n'importe quelle substance susceptible de fixer de façon covalente ou non, directement ou non, le premier anticorps. La fixation peut s'effectuer par adsorption, par liaison chimique covalente ou par l'intermédiaire de molécules liantes telles que des anticorps et le système avidine-biotine. On peut aussi utiliser des phases solides en matières minérales comme l'hydroxylapatite, la mullite, l'alumine, $ZrO_2$ et $Ca_3(PO_4)_2$.

La fixation du premier anticorps sur la surface de la phase solide peut être passive ou active. Elle peut être obtenue par adsorption directe ou au moyen de réactifs appropriés.

Dans le procédé de l'invention, le premier et le second anticorps utilisés sont tous deux des anticorps spécifiques de la substance à doser.

Ce premier et ce second anticorps peuvent provenir de la même source ou de sources différentes. De plus, ces anticorps peuvent être des anticorps polyclonaux ou des anticorps monoclonaux.

Dans le cas où la substance à doser est un haptène monoépitopique, le premier et le second anticorps sont identiques et de préférence monoclonaux.

Dans le cas où la substance à doser est un antigène, on peut utiliser deux anticorps différents, mais il est avantageux d'utiliser deux anticorps identiques, et de préférence également des anticorps monoclonaux. Pour le second anticorps marqué, le marquage peut être réalisé au moyen de diverses techniques, par exemple au moyen d'un élément radioactif, d'une enzyme, d'un marqueur fluorescent, d'un marqueur luminescent, ou de molécules capables de réagir avec l'avidine ou la streptavidine comme la biotine et ses analogues structuraux.

Lorsque le second anticorps est marqué au moyen d'une molécule telle qu'une enzyme, un marqueur fluorescent, un marqueur luminescent ou une molécule capable de réagir avec l'avidine ou la streptavidine, le couplage du marqueur avec l'anticorps peut être réalisé par les techniques classiques utilisées habituellement dans ce type de dosage.

Le procédé de l'invention s'applique avantageusement au dosage des antigènes car, dans ce cas, on peut obtenir la même sensibilité qu'avec les dosages de type "sandwich" en utilisant un seul anticorps, de préférence un anticorps monoclonal.

Toutefois, le procédé de l'invention présente un intérêt encore plus important pour le dosage des haptènes monoépitopiques car il permet d'obtenir dans ce cas des sensibilités beaucoup plus grandes que dans les dosages par compétition, en utilisant un seul anticorps, mettant à profit l'utilisation de réactifs en excès (anticorps de capture et anticorps marqués) comme cela est fait dans les dosages immunométriques à deux sites.

A titre d'exemple d'haptènes susceptibles d'être dosés par le procédé de l'invention, on peut citer l'ACTH, l'angiotensine, l'ANF, la bradykinine, l'enképhaline, le LHRH, l'ocytocine, la vasopressine, les neurokinines, l'endothéline, la substance P, la thyroxine et le leucotriène $E_4$.

Les formules correspondantes de ces haptènes sont données ci-après.

```
ACTH(18-39)    :    Arg-Pro-Val-Lys-Val-Tyr-Pro-Asn-Gly-
Ala-Glu-Asp-Glu-Ser-Ala-Glu-Ala-Phe-Pro-Leu-Glu-
Phe

ANGIOTENSINE II : Asp-Arg-Val-Tyr-Ile-His-Pro-Phe

ANF(1-28)    :    Ser-Leu-Arg-Arg-Ser-Ser-Cys-Phe-Gly-
Gly-Arg-Met-Asp-Arg-Ile-Gly-Ala-Gln-Ser-Gly-Leu-
Gly-Cys-Asn-Ser-Phe-Arg-Tyr

BRADYKININE : Arg-Pro-Pro-Gly-Phe-Ser-Pro-Phe-Arg

ENKEPHALINE : Tyr-Gly-Gly-Phe-Met
```

LHRH : Pyr-His-Trp-Ser-Tyr-Gly-Leu-Arg-Pro-Gly-$NH_2$

```
OCYTOCINE    :    Cys-Tyr-Ile-Gln-Asn-Cys-Pro-Leu-Gly-
```
$NH_2$

VASOPRESSINE    :    Cys-Tyr-Phe-Gln-Asn-Cys-Cys-Pro-Arg-
Gly-$NH_2$

NEUROKININE    A    :    His-Lys-Thr-Asp-Ser-Phe-Val-Gly-
Leu-Met-$NH_2$

NEUROKININE    B    :    Asp-Met-His-Asp-Phe-Phe-Val-Gly-
Leu-Met-$NH_2$

```
ENDOTHELINE :    cis-Ser-cis-Ser-Ser-Leu-Met-Asp-Lys-
Glu-cis-Val-Tyr-Phe-cis-His-Leu-Asp-Ile-Ile-Trp
```

SUBSTANCE    P    :    Arg-Pro-Lys-Pro-Gln-Gln-Phe-Phe-Gly-
Leu-Met-$NH_2$

THYROXINE :

D'autres caractéristiques et avantages de l'invention apparaîtront mieux à la lecture de la description qui suit, donnée bien entendu à titre illustratif et non limitatif, en référence aux dessins annexés, sur lesquels

- les figures 1A à 1E sont des représentations schématiques des différentes étapes du procédé de dosage immunométrique de l'invention,
- la figure 2 est une courbe d'étalonnage obtenue pour le dosage immunométrique de la substance P par le procédé de l'invention,
- la figure 3 est une courbe d'étalonnage obtenue pour le dosage par compétition de la substance P par un procédé conforme à l'art antérieur,
- la figure 4 est un diagramme illustrant les profils de précision obtenus pour la substance P avec le dosage immunométrique de l'invention et avec le dosage par compétition de l'art antérieur,
- la figure 5 est un diagramme illustrant les sensibilités des dosages de la substance P par le procédé de l'invention et par le procédé de l'art antérieur,
- la figure 6 est un diagramme illustrant l'influence de la concentration en glutaraldéhyde sur le dosage de la substance P conformément au procédé de l'invention,

- la figure 7 est un diagramme illustrant l'influence du pH de l'étape d'immobilisation sur le dosage de la substance P conformément au procédé de l'invention,
- la figure 8 est un diagramme illustrant l'influence des différentes étapes du procédé de l'invention sur le dosage de la substance P,
- la figure 9 est une courbe d'étalonnage obtenue pour le dosage de la thyroxine conformément au procédé de l'invention,
- la figure 10 représente les courbes d'étalonnage obtenues pour des dosages de la thyroxine selon le procédé de l'invention, et
- la figure 11 est un diagramme illustrant l'influence des différentes étapes du procédé de l'invention sur le dosage de la thyroxine.

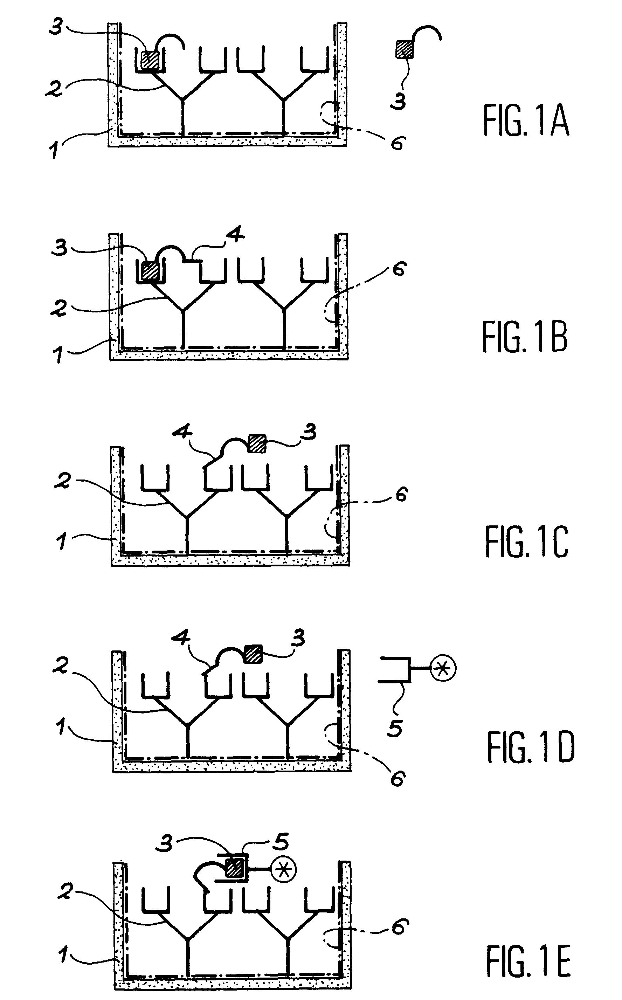

Sur les figures 1A à 1E, on a représenté les différentes étapes du procédé de dosage immunométrique de l'invention.

Ainsi, sur la figure 1A qui représente la première étape, on voit une phase solide (1) sur laquelle sont fixés un anticorps de capture (2) spécifique de la substance à doser (3) et la substance de saturation (6) constituée généralement par des protéines comme la sérumalbumine bovine. Pour effectuer cette étape, on a mis en contact le support (1) muni des anticorps de capture (2) et de la substance de saturation (6) avec la substance à doser (3) pour lier immunologiquement cette substance (3) avec l'anticorps (2) fixé. La quantité d'anticorps fixée sur la phase solide correspond à un excès par rapport à la quantité de substance à doser présente dans l'échantillon.

Dans la deuxième étape, dont les deux stades sont représentés sur les figures 1B et 1C, on soumet la phase solide à un traitement au moyen d'un réactif au moins bifonctionnel pour immobiliser la substance à doser (3) par formation d'une liaison covalente (4) sur la phase solide (1) (figure 1B). Cette liaison covalente peut être établie directement avec la phase solide, avec le premier anticorps de capture et/ou avec la substance de saturation.

Dans le second stade, on rend accessible l'épitope de la substance à doser (3) en vue d'une nouvelle réaction immunologique en dénaturant sa liaison immunologique avec l'anticorps (2).

Pour effectuer le premier stade d'immobilisation, on met en contact la phase solide (1) sur laquelle sont fixés la substance de saturation, l'anticorps de capture (2) et la substance à doser (3) liée immunologiquement à cet anticorps, avec une solution d'un réactif au moins bifonctionnel, à un pH approprié, sous agitation, pendant une durée suffisante.

Le pH dépend en particulier du réactif polyfonctionnel utilisé. Ainsi, dans le cas du glutaraldéhyde, on préfère un pH de 5 à 9, alors que dans le cas du disuccinimidyl subérate (DSS), on préfère un pH de 7 à 9. Ces pH sont obtenus au moyen de tampons appropriés.

Pour arrêter la réaction, on peut ensuite ajouter une solution d'arrêt qui a pour rôle, soit de détruire les groupements fonctionnels existants capables de réagir avec le réactif bifonctionnel, soit de réagir avec les groupements fonctionnels libres du réactif bifonctionnel. Ainsi, lorsqu'on utilise comme réactif bifonctionnel le glutaraldéhyde, la solution d'arrêt peut comprendre un agent réducteur tel que $NaBH_4$, pour détruire les fonctions aldéhydes en excès du glutaraldéhyde, ou une amine, telle que l'éthanol amine pour utiliser les groupements restants du glutaraldéhyde capables de réagir avec les fonctions amines.

Dans le second stade (fig. 1C), on effectue un traitement de dénaturation de la liaison immunologique de la substance à doser avec le premier anticorps.

Ce traitement de dénaturation peut être effectué de façon classique au moyen de réactifs appropriés, ou encore sous l'action des ultrasons ou de la chaleur.

Ces réactifs peuvent être choisis, par exemple, parmi les acides comme HCl, les bases comme NaOH, les solvants organiques, par exemple les alcools tels que le méthanol, les agents tensioactifs et les sels minéraux. Le réactif utilisé est choisi en fonction du couple antigène-anticorps et de la nature de la liaison entre le premier anticorps et la phase solide.

Dans certains cas, il n'est pas nécessaire d'effectuer ce second stade de dénaturation, car cette dénaturation peut être obtenue simultanément dans le premier stade d'immobilisation, en raison des conditions réactionnelles utilisées.

Dans la troisième étape représentée sur la figure 1D, on met en contact la phase solide (1) sur laquelle est immobilisée la substance à doser (3) par une liaison covalente (4) avec un second anticorps marqué (5) spécifique de la substance (3). Ceci est effectué au moyen d'une solution contenant une quantité d'anticorps marqué correspondant à un excès par rapport à la quantité de substance à doser immobilisée.

Dans la quatrième étape (figure 1E), on mesure la quantité d'anticorps marqué (5) fixée sur la phase solide par liaison immunologique avec la substance à doser (3). Cette mesure est effectuée par des méthodes classiques telles que celles mises en oeuvre habituellement pour ce type de dosage, selon le marqueur utilisé.

Pour obtenir la courbe d'étalonnage, on effectue plusieurs dosages sur des solutions standards ayant des concentrations connues en substance à doser, en opérant dans les mêmes conditions, ce qui donne les taux d'anticorps fixés correspondant aux différentes concentrations en vue d'établir cette courbe.

On effectue ensuite le dosage d'un échantillon de concentration inconnue dans les mêmes conditions, et en se reportant à la courbe d'étalonnage, on obtient à partir de la mesure de la quantité de second anticorps fixée, la concentration en substance à doser de l'échantillon.

On précise que chaque étape de ce procédé est précédée par une opération de lavage classique, effectuée au moyen de tampons de lavage.

On décrit, ci-après, des dosages réalisés conformément à ce procédé.

**Exemple 1 :** Dosage de la substance P.

Dans cet exemple, on utilise comme phase solide une plaque de microtitration à 96 puits en polystyrène, sur laquelle on fixe un anticorps monoclonal antisubstance P, constitué par l'anticorps SP14 décrit par Couraud et al dans J. of Neurochemistry, vol 49, n°6, 1987, p. 1708-1718, en opérant de la façon suivante.

On ajoute dans chaque puits 200µl de tampon phosphate $5.10^{-2}M$ (pH 7,4) contenant 10µg/ml d'anticorps antisubstance P (SP14) et on laisse incuber pendant 18h à 22°C. On lave ensuite chaque puits avec un tampon de lavage constitué par le tampon phosphate utilisé précédemment qui contient en plus 0,05% de Tween 20, puis on ajoute dans chaque puits 300µl du tampon EIA qui est un tampon au phosphate de potassium 0,1M (pH 7,5) contenant 0,1mol/l de NaCl, 1mmol/l d'acide éthylène diamine tétracétique (EDTA), 0,1% de sérum albumine bovine (BSA) et 0,01% d'azoture de sodium, pour saturer la phase solide avec une substance de saturation (BSA), et on maintient les plaques à 4°C pendant au moins 24h avant la première utilisation.

On réalise ensuite la première étape du dosage en introduisant dans chaque puits, après lavage par le tampon de lavage, une solution standard de substance P ou l'échantillon, à raison de 100µl dans le tampon EIA, on laisse incuber pendant 18h à +4°C, puis on procède à un lavage au moyen du tampon de lavage.

On réalise ensuite la deuxième étape en deux stades. Dans le premier stade, on ajoute dans chaque puits 100µl de tampon borate 0,1M (pH 9) contenant 2,5% de glutaraldéhyde et on laisse réagir pendant 1h sous agitation modérée. Après lavage, on ajoute dans chaque puits 250µl d'une solution d'arrêt constituée par une solution de borohydrure de sodium à 4mg/ml, et on laisse réagir pendant 1h, puis on procède à un lavage par le tampon de lavage.

Dans le second stade, on ajoute 250µl de HCl 0,1N et on laisse réagir pendant 10min à 22°C, ce qui conduit à dénaturer la liaison immunologique entre la substance P (3) et le premier anticorps (2) pour rendre accessible l'épitope de la substance P en vue d'une nouvelle réaction immunologique.

Après lavage, on réalise la troisième étape en ajoutant dans chaque puits 200µl de l'anticorps monoclonal antisubstance P utilisé dans la première étape qui a été marqué avec de l'acétylcholinestérase (AChE) (5EU/ml), et qui est dilué dans le tampon EIA, et on laisse incuber pendant 18h à 4°C. On procède ensuite à un lavage.

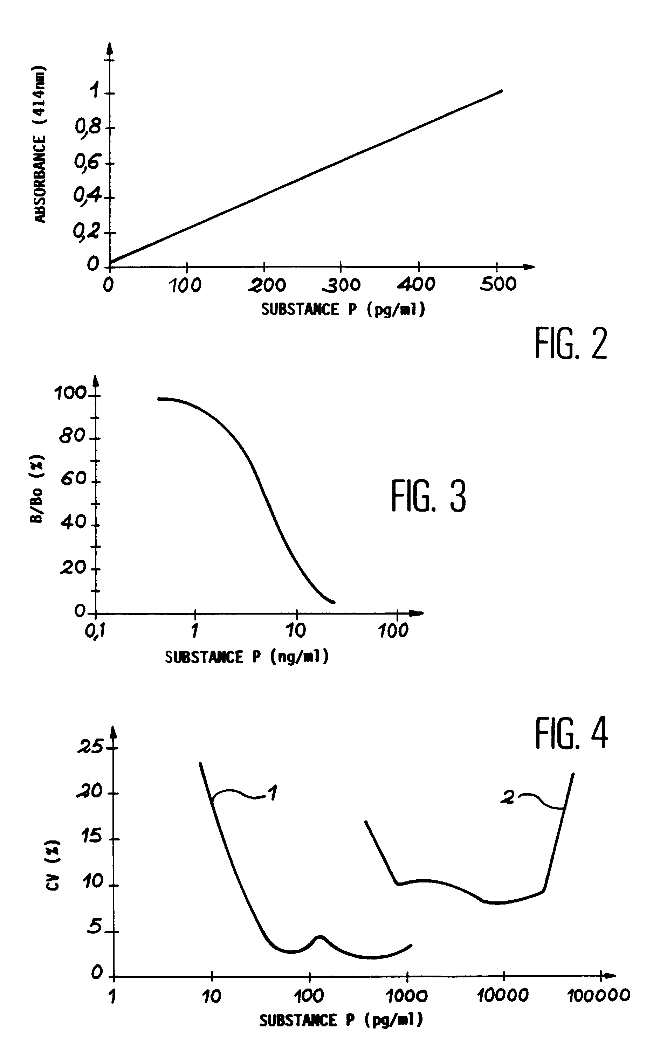

Pour réaliser la quatrième étape, c'est-à-dire mesurer la quantité du second anticorps (5) fixé sur la phase solide, on mesure l'activité enzymatique en ajoutant dans chaque puits 200µl de réactif d'Ellman, constitué par un mélange d'acétyl thiocholine et de DTNB comme décrit par Pradelles et al dans Anal. Chem. 57, 1985, p. 1170, en réalisant la réaction enzymatique pendant 1h et en déterminant ensuite l'absorbance à 414nm. A partir des mesures d'absorbance obtenues pour les solutions standard, on trace la courbe d'étalonnage, représentée sur la figure 2, qui donne les concentrations en substance P (en pg/ml) en fonction de l'absorbance à 414nm.

A partir de l'absorbance obtenue dans le puits contenant l'échantillon, on peut déterminer la concentration en substance P de l'échantillon en se reportant à la courbe d'étalonnage de la figure 2.

Ainsi, le procédé de l'invention permet un dosage précis et sensible de la substance P, la concentration détectable étant de 6pg/ml.

**Exemple comparatif 1** : Dosage de la substance P.

Dans cet exemple, on utilise la technique du dosage par compétition et le même anticorps monoclonal SP14 que dans l'exemple 1 pour doser la substance P.

Dans ce cas, on utilise une plaque de microtitration et on utilise l'anticorps monoclonal SP 14 en opérant comme décrit par Couraud et al dans J. of Neurochemistry, vol 49, n° 6, 1987, p. 1708-1718, puis on introduit dans chaque puits 50µl de solution standard ou d'échantillon et 50µl du conjugué substance P-acétylcholinestérase servant de traceur enzymatique. Après incubation pendant une nuit à 4°C, on lave les plaques et on détermine l'activité enzymatique immobilisée en opérant comme dans l'exemple 1.

On obtient ainsi la courbe d'étalonnage représentée sur la figure 3, sur laquelle on a indiqué en ordonnée le rapport $B/B_0$ (en %) en fonction de la concentration en substance P (en ng/ml). On précise que B représente l'absorbance mesurée en présence de substance P et $B_0$ représente l'absorbance mesurée en l'absence de substance P.

Sur la figure 4, on a illustré les profils de précision obtenus dans le dosage de l'exemple 1 (courbe 1) et le dosage de l'exemple comparatif 1 (courbe 2).

Ces profils de précision représentent les variations du coefficient de précision CV (en %) en fonction de la quantité de substance P (en pg/ml) en coordonnée logarithmique.

Pour tracer ces courbes, on effectue 8 mesures pour chaque concentration de substance P dans la solution standard afin de déterminer la déviation standard d et la valeur moyenne $v_m$ relative à chaque concentration. Le coefficient de précision CV (en %) est évalué à partir de ces mesures par la formule :

$$CV = \frac{d}{v_m} \times 100$$

Sur la figure 4, on remarque ainsi que le coefficient de précision et la sensibilité sont meilleurs dans le cas de la courbe 1, et que le coefficient de précision est en particulier très bon dans la gamme de concentrations allant de 30 à 1000pg/ml.

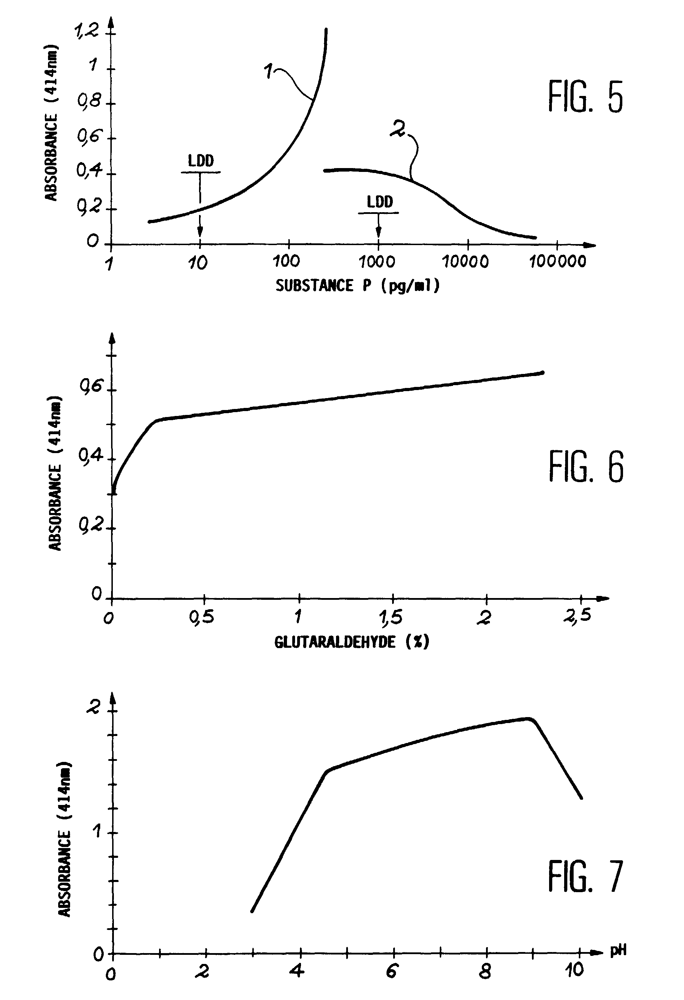

Sur la figure 5, on a représenté l'absorbance en fonction de la concentration en substance P dans le cas du dosage de l'exemple 1 (courbe 1) et dans le cas du dosage de l'exemple comparatif 1 (courbe 2).

Au vu de ces résultats, on remarque que la limite de détection (LDD) est de l'ordre de 10 pg/ml dans le cas du dosage de l'invention, alors qu'elle est de l'ordre de 900 pg/ml dans le cas du dosage par compétition conforme à l'art antérieur.

Ainsi, le procédé de dosage immunométrique de l'invention permet un dosage précis et sensible de la substance P. Il permet notamment d'atteindre une plus grande précision et de détecter des quantités bien moindres de substance P.

**Exemple 2** : Dosage de la substance P.

Dans cet exemple, on étudie l'influence de la concentration en glutaraldéhyde utilisé dans la deuxième étape d'immobilisation de la substance P sur les résultats du dosage. Dans ce cas, on effectue des dosages comme dans l'exemple 1 sur une solution standard contenant 500pg/ml de substance P en utilisant des concentrations en glutaraldéhyde qui varient de 0 à 2,5%, et on mesure dans chaque cas, l'absorbance à 414nm.

Les résultats obtenus sont donnés sur la figure 6 qui représente l'absorbance à 414nm en fonction de la concentration en glutaraldéhyde.

On remarque ainsi que l'absorbance augmente avec la concentration en glutaraldéhyde, et que de bons résultats sont obtenus dans la gamme de concentrations allant de 0,5 à 2,5%.

**Exemple 3** : Dosage de la substance P.

Dans cet exemple, on étudie l'influence du pH utilisé lors de l'étape d'immobilisation, c'est-à-dire du pH de la solution de glutaraldéhyde sur le dosage.

Dans ce cas, on suit le même mode opératoire que dans l'exemple 1 sur une solution standard à 500pg/ml de substance P en utilisant lors de l'étape d'immobilisation des pH allant de 3 à 10.

La figure 7 représente l'évolution de l'absorbance en fonction du pH utilisé. On remarque ainsi que l'absorbance est meilleure dans la gamme de pH allant de 5 à 9.

**Exemples 4 à 7** : Dosage de la substance P.

Dans ces exemples, on étudie l'influence des deux stades de la deuxième étape du procédé de l'invention sur le dosage.

Dans tous les exemples, on suit le même mode opératoire que dans l'exemple 1, mis à part les modifications suivantes de la deuxième étape.

Dans l'exemple 4, on supprime l'utilisation de NaBH$_4$, c'est-à-dire qu'on n'utilise pas de solution d'arrêt de la réaction d'immobilisation.

Dans l'exemple 5, on n'utilise pas de solution de glutaraldéhyde, mais on effectue l'étape de réduction par NaBH$_4$.

Dans l'exemple 6, on supprime complètement le stade d'immobilisation, c'est-à-dire la réaction avec la solution de glutaraldéhyde et l'addition de la solution d'arrêt.

Dans l'exemple 7, on effectue le stade d'immobilisation et pas le stade de libération de l'épitope par la solution d'acide chlorhydrique.

Ces modifications sont regroupées dans le tableau qui suit.

Tableau 1

| Etape 2 | | Ex 1 | Ex 4 | Ex 5 | Ex 6 | Ex 7 |
|---|---|---|---|---|---|---|
| Stade 1 | glutaraldéhyde | oui | oui | non | non | oui |
| | NaBH$_4$ | oui | non | oui | non | oui |
| Stade 2 | HCl | oui | oui | oui | oui | non |

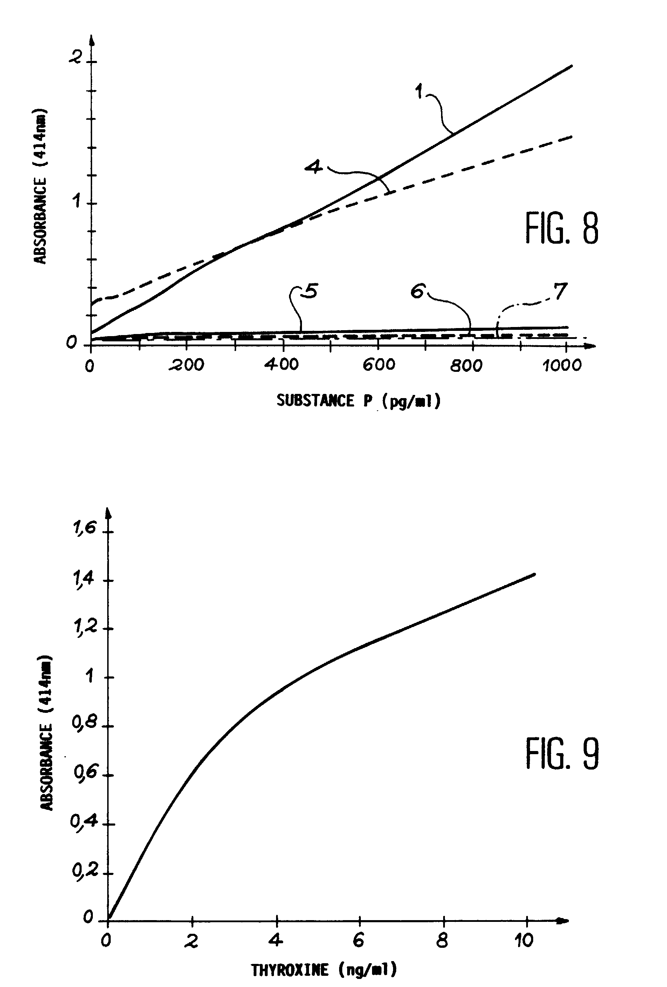

Les résultats obtenus sont donnés sur la figure 8 qui représente les courbes d'étalonnage obtenues dans chaque cas, soit l'évolution de l'absorbance à 414nm en fonction de la concentration en substance P (pg/ml).

Sur cette figure, la courbe 1 se réfère à l'exemple 1, la courbe 4 se réfère à l'exemple 4, La courbe 5 se réfère à l'exemple 5, la courbe 6 se réfère à l'exemple 6 et la courbe 7 se réfère à l'exemple 7.

Au vu de cette figure, on remarque que la réalisation des stades d'immobilisation de la substance P sur les plaques de microtitration et de libération de son épitope est essentielle pour permettre le dosage de la substance P.

**Exemple 8** : Dosage de la thyroxine.

Pour ce dosage, on utilise une plaque de microtitration à 96 puits identique à celle de l'exemple 1 et on fixe dans les puits un anticorps antithyroxine constitué par un anticorps monoclonal de l'Institut Pasteur, en opérant dans les mêmes conditions que celles de l'exemple 1.

On introduit ensuite dans chaque puits, une solution standard de thyroxine à raison de 100µl dans un tampon barbital 0,05M (pH 8,6) contenant 0,1% de BSA, puis on laisse incuber pendant 1h à 22°C. On lave ensuite au moyen de tampon de lavage utilisé dans l'exemple 1.

Dans la seconde étape, on ajoute dans chaque puits, 100µl de tampon phosphate 0,1M (pH 7) et 10µl de disuccinimidyl subérate (DSS) à une concentration de 1mg/ml dans un mélange de diméthylformamide et de n-propanol (1/9 en volume), et on laisse réagir pendant 15min à la température de 22°C, puis on lave avec le tampon de lavage et on arrête la réaction avec de l'éthanolamine 0,1M dans le tampon borate 0,1M (pH9). Après lavage, on ajoute 250µl de soude 0,1N dans chaque puits pour libérer l'épitope de la thyroxine.

Après lavage, on ajoute 100µl du même anticorps monoclonal anti-thyroxine marqué à l'acétylcholinestérase (AChE) (5EU/ml) dilué dans le tampon barbital et on laisse incuber pendant 1h à 22°C.

Après lavage, on ajoute le réactif d'Ellman (200µl), on laisse la réaction enzymatique se dérouler pendant 10min, puis on mesure l'absorbance à 414nm.

Les résultats obtenus, c'est-à-dire la courbe d'étalonnage, sont donnés sur la figure 9 qui représente l'évolution de l'absorbance à 414nm en fonction de la concentration en thyroxine (ng/ml).

La limite de détection est de 61pg/ml.

On obtient donc une sensibilité élevée en utilisant un seul anticorps et en améliorant la spécificité du dosage par l'utilisation d'un anticorps monoclonal.

A titre comparatif, on précise que le même dosage effectué par compétition avec le même anticorps donne une limite de détection de 1ng/ml.

**Exemples 9 à 11:** Dosages de la thyroxine.

Pour ces dosages, on suit le même mode opératoire que dans l'exemple 8 en utilisant différents agents pour inhiber l'action des protéines liantes comme les TBG, et en ajoutant 10µl de plasma standard ou d'échantillon, soit à 100µl de tampon barbital (exemple 9), soit à 100µl de tampon barbital contenant 1mg/ml d'ANS (acide 8-anilino-2-naphtalène sulfonique) (exemple 10), soit à 100µl de tampon barbital contenant 0,4% de thymérosal (exemple 11).

On réalise ensuite l'étape d'immobilisation de la thyroxine dans les puits en utilisant une solution de glutaraldéhyde à 2,5% dans le tampon borate, que l'on fait réagir pendant 30min sous agitation modérée, puis on arrête la réaction au moyen de NaBH$_4$ que l'on fait réagir pendant 30min.

Pour la libération de l'épitope et la suite des étapes de dosage, on procède comme dans l'exemple 8.

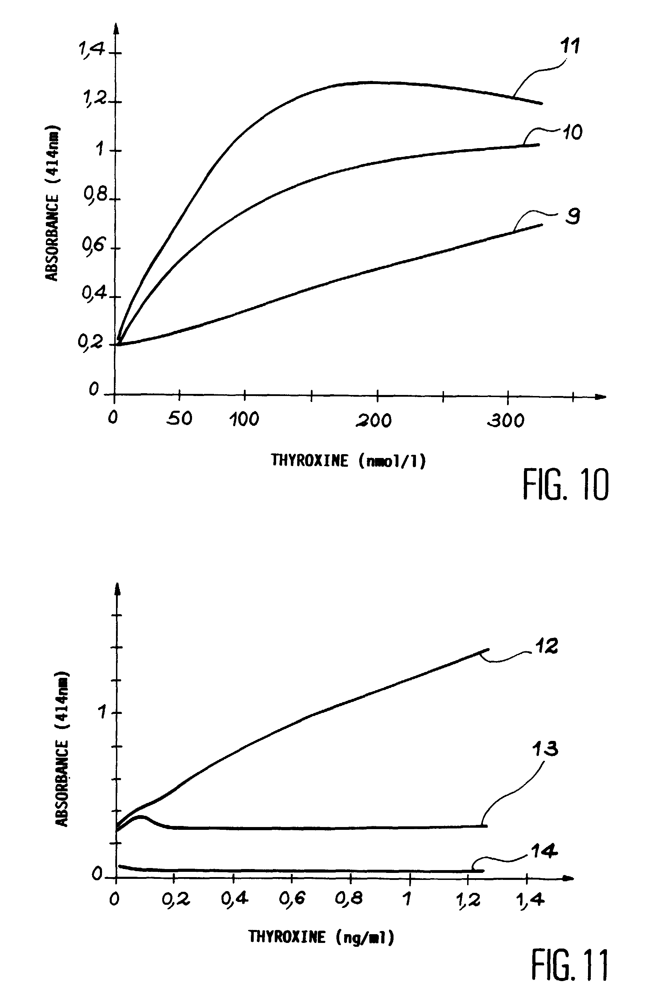

Les résultats obtenus sont donnés sur la figure 10 qui représente les courbes d'étalonnage obtenues, soit l'absorbance à 414nm en fonction de la concentration en thyroxine (en nmol/l).

Sur cette figure, la courbe 9 se réfère à l'exemple 9, la courbe 10 se réfère à l'exemple 10 et la courbe 11 se réfère à l'exemple 11.

Au vu de cette figure, on remarque que les meilleurs résultats sont obtenus lorsqu'on utilise le tampon barbital avec du thymérosal.

**Exemples 12 à 14** : <u>Dosages de la thyroxine.</u>

Dans ces exemples, on étudie l'influence des différentes étapes du procédé de l'invention sur le dosage.

Dans tous les exemples, on suit le même mode opératoire que dans l'exemple 8 mises à part les modifications suivantes.

Dans l'exemple 12, on utilise le DSS comme réactif d'immobilisation, de l'éthanolamine comme solution d'arrêt mais de l'acide chlorhydrique 0,1N comme réactif de libération de l'épitope.

Dans l'exemple 13, on effectue uniquement l'étape d'immobilisation de la thyroxine par le DSS et l'éthanolamine mais sans libération de l'épitope par HCl.

Dans l'exemple 14, on n'effectue ni l'étape d'immobilisation de la thyroxine, ni l'étape de libération de l'épitope.

Les résultats obtenus sont représentés sur la figure 11 qui représente les courbes d'étalonnage obtenues. Sur cette figure, les courbes 12, 13 et 14 se réfèrent respectivement aux exemples 12 à 14.

Au vu de cette figure, on remarque que les deux étapes d'immobilisation et de libération de l'épitope sont essentielles pour permettre le dosage de la thyroxine puisqu'on ne remarque aucune variation d'absorbance en fonction de la concentration en thyroxine dans le cas des exemples 13 et 14.

Le procédé de l'invention est donc très intéressant car il permet d'obtenir une sensibilité élevée, en utilisant un seul anticorps. Par ailleurs, les résultats donnés dans le tableau 2, ci-dessous, d'études de corrélation effectuées sur des substance P provenant de différentes sources, ont montré qu'on obtenait avec le procédé de l'invention des résultats équivalents à ceux d'un dosage par compétition au moyen du même anticorps, ce qui confirme la fiabilité du procédé de l'invention.

Tableau 2

| Origine de la Substance P | Dosage de l'invention SP (ng/ml) | Dosage par compétition SP (ng/ml) |
|---|---|---|
| Extrait de Cerveau de rat | 70 | 75,5 |
| Extrait de Moëlle épinière de rat | 61,4 | 61,3 |
| Extrait de Cerveau de souris | 75,5 | 70 |
| Extrait de Moëlle épinière de souris | 33,4 | 32,1 |

## Revendications

1.  Procédé de dosage immunométrique d'une substance constituée par un antigène ou un haptène, caractérisé en ce qu'il comprend les étapes suivantes :

    1°) mettre en contact un échantillon contenant la substance à doser avec une phase solide sur laquelle sont fixés une substance de saturation et un anticorps de capture, dit premier anticorps, spécifique d'un épitope de la substance à doser, pour lier immunologiquement la substance à doser avec l'anticorps fixé,

    2°) soumettre la phase solide sur laquelle sont fixés la substance de saturation, l'anticorps et la substance à doser à un traitement au moyen d'au moins un réactif pour immobiliser la substance à doser sur la phase solide par formation de liaisons covalentes et pour dénaturer la liaison immunologique obtenue précédemment entre la substance à doser et le premier anticorps afin de rendre accessible un épitope de la substance à doser en vue d'une nouvelle réaction immunologique,

    3°) mettre en contact la phase solide ainsi traitée avec un anticorps marqué, dit second anticorps, spécifique de la substance à doser,

    4°) mesurer la quantité du second anticorps fixé sur la phase solide, et

    5°) déterminer sur une courbe d'étalonnage la quantité de substance à doser présente dans l'échantillon à partir de la quantité du second anticorps mesurée dans la quatrième étape.

2.  Procédé selon la revendication 1, caractérisé en ce que, dans la deuxième étape, on fait réagir la substance à doser liée immunologiquement au premier anticorps, avec un réactif au moins bifonctionnel capable de former des liaisons covalentes, d'une part avec la substance à doser, et, d'autre part, avec la phase solide revêtue du premier anticorps et de la substance de saturation pour immobiliser la substance à doser sur la phase solide au moyen de ce réactif.

3.  Procédé selon la revendication 2, caractérisé en ce que le réactif est le glutaraldéhyde ou le disuccinimidyl subérate.

**4.** Procédé selon l'une quelconque des revendications 1 à 3, caractérisé en ce que la deuxième étape comprend un traitement de dénaturation effectué au moyen d'un réactif.

**5.** Procédé selon la revendication 4, caractérisé en ce que le réactif est choisi parmi les acides, les bases, les solvants organiques, les agents tensioactifs et les sels minéraux.

**6.** Procédé selon l'une quelconque des revendications 1 à 3, caractérisé en ce que la deuxième étape comprend un traitement de dénaturation effectué par action d'ultrasons ou par action de la chaleur.

**7.** Procédé selon l'une quelconque des revendications 1 à 6, caractérisé en ce que le premier et le second anticorps sont identiques.

**8.** Procédé selon l'une quelconque des revendications 1 à 7, caractérisé en ce que les deux anticorps sont des anticorps monoclonaux.

**9.** Procédé selon l'une quelconque des revendications 1 à 8, caractérisé en ce que le second anticorps est marqué au moyen d'un élément radioactif, d'une enzyme, d'un marqueur fluorescent, d'un marqueur luminescent ou d'une molécule capable de réagir avec l'avidine ou la streptavidine.

**10.** Procédé selon l'une quelconque des revendications 1 à 9, caractérisé en ce que la subtance à doser est la substance P ou la thyroxine.

**11.** Procédé selon l'une quelconque des revendications 1 à 9, caractérisé en ce que la substance à doser est un haptène choisi dans le groupe comprenant l'ACTH, l'angiotensine, l'ANF, la bradykinine, l'enképhaline, le LHRH, l'ocytocine, la vasopressine, les neurokinines, l'endothéline et le leucotriène $E_4$.

## Claims

**1.** Process for the immunometric determination of a substance constituted by an antigen or a hapten, characterized in that it comprises the following stages:

1) contacting a sample containing the substance to be determined with a solid phase on which are fixed a saturation substance and a capture antibody, called the first antibody, which is specific to an epitope of the substance to be determined, in order to immunologically bond the substance to be determined to the fixed antibody,
2) subjecting the solid phase on which are fixed the saturation substance the antibody and the substance to be determined to a treatment by means of at least one reagent in order to immobilize the substance to be determined on the solid phase by the formation of covalent bonds and for denaturing the immunological bond previously obtained between the substance to be dosed and the first antibody in order to make accessible an epitope of the substance to be determined with a view to a further immunological reaction,
3) contacting the thus treated solid phase with a labelled antibody, called the second antibody, which is specific to the substance to be determined,
4) measuring the quantity of the second antibody fixed to the solid phase and
5) determining on a calibration curve the quantity of the substance to be determined which is present in the sample on the basis of the quantity of the second antibody measured in the fourth stage.

**2.** Process according to claim 1, characterized in that, in the second stage, the substance to be determined immunologically bonded with the first antibody, is reacted with an at least bifunctional reagent able to form covalent bonds on the one hand with the substance to be determined and on the other with the solid phase coated with the first antibody and the saturation substance in order to immobilize the substance to be determined on the solid phase by means of said reagent.

**3.** Process according to claim 2, characterized in that the reagent is glutaraldehyde or disuccinimidyl suberate.

**4.** Process according to any one of the claims 1 to 3, characterized in that the second stage comprises a denaturation treatment performed by means of a reagent.

5. Process according to claim 4, characterized in that the reagent is chosen from among acids, bases, organic solvents, surfactants and mineral salts.

6. Process according to any one of the claims 1 to 3, characterized in that the second stage comprises a denaturation treatement performed by the action of ultrasonics or heat.

7. Process according to any one of the claims 1 to 6, characterized in that the first and second antibodies are identical.

8. Process according to any one of the claims 1 to 7, characterized in that the two antibodies are monoclonal antibodies.

9. Process according to any one of the claims 1 to 8, characterized in that the second antibody is marked by means of a radioactive element, an enzyme, a fluorescent marker, a luminescent marker or a molecule able to react with avidin or streptavidin.

10. Process according to any one of the claims 1 to 9, characterized in that the substance to be determined is substance P or thyroxine.

11. Process according to any one of the claims 1 to 9, characterized in that the substance to be determined is a hapten chosen from within the group including ACTH, angiotensin, ANF, bradykinin, encephalin, LHRH, oxytocin, vasopressin, neurokinins, endotheline and leucotriene $E_4$.

**Patentansprüche**

1. Verfahren zur immunologischen Bestimmung einer Substanz, die aus einem Antigen oder einem Hapten besteht, dadurch gekennzeichnet, daß sie die folgenden Schritte umfaßt:

1°) eine Probe, welche die zu bestimmende Substanz enthält, mit einer festen Phase in Kontakt zu bringen, auf welcher eine Sättigungssubstanz und ein Fang-Antikörper, erster Antikörper genannt, der für ein Epitop der zu bestimmenden Substanz spezifisch ist, fixiert sind, um die zu bestimmende Substanz immunologisch mit dem fixierten Antikörper zu verbinden,

2°) die feste Phase, auf welcher die Sättigungssubstanz, der Antikörper und die zu bestimmende Substanz fixiert sind, einer Behandlung durch zumindest ein Reagens zu unterziehen, um die zu bestimmende Substanz durch Bildung von kovalenten Bindungen auf der festen Phase zu immobilisieren und um die vorher erhaltene immunologische Bindung zwischen der zu bestimmenden Substanz und dem ersten Antikörper zu denaturieren, damit ein Epitop der zu bestimmenden Substanz im Hinblick auf eine neue immunologische Reaktion zugänglich gemacht wird,

3°) die so behandelte feste Phase mit einem markierten Antikörper, zweiter Antikörper genannt, der spezifisch ist für die zu bestimmende Substanz, in Kontakt zu bringen,

4°) die auf der festen Phase fixierte Menge des zweiten Antikörpers zu messen,

5°) aus der im vierten Schritt gemessenen Menge des zweiten Antikörpers auf einer Eichkurve die in der Probe vorhandene Menge der zu bestimmenden Substanz zu ermitteln.

2. Verfahren nach Anspruch 1, dadurch gekennzeichnet, daß man die zu bestimmende, immunologisch an den ersten Antikörper gebundene Substanz in dem zweiten Schritt mit einem zumindest bifunktionellen Reagens reagieren läßt, das imstande ist, einerseits mit der zu bestimmenden Substanz, andererseits mit der festen, von dem ersten Antikörper und der Sättigungssubstanz überzogenen Phase kovalente Bindungen zu bilden, um mit Hilfe dieses Reagens die zu bestimmende Substanz auf der festen Phase zu immobilisieren.

3. Verfahren nach Anspruch 2, dadurch gekennzeichnet, daß das Reagens Glutaraldehyd oder Disuccinimidylsuberat ist.

4. Verfahren nach einem der Ansprüche 1 bis 3, dadurch gekennzeichnet, daß der zweite Schritt eine Denaturierungsbehandlung umfaßt, die mit einem Reagens ausgeführt wird.

5. Verfahren nach Anspruch 4, dadurch gekennzeichnet, daß das Reagens unter den Säuren, den Basen, den organischen Lösungsmitteln, den Detergentien und den mineralischen Salzen gewählt wird.

6. Verfahren nach einem der Ansprüche 1 bis 3, dadurch gekennzeichnet, daß der zweite Schritt eine Denaturierungsbehandlung umfaßt, die durch Einwirkung von Ultraschall oder durch Einwirkung von Wärme ausgeführt wird.

7. Verfahren nach einem der Ansprüche 1 bis 6, dadurch gekennzeichnet, daß der erste und der zweite Antikörper identisch sind.

8. Verfahren nach einem der Ansprüche 1 bis 7, dadurch gekennzeichnet, daß die beiden Antikörper monoklonale Antikörper sind.

9. Verfahren nach einem der Ansprüche 1 bis 8, dadurch gekennzeichnet, daß der zweite Antikörper mit einem radioaktiven Element, einem Enzym, einem fluoreszierenden Marker, einem lumineszierenden Marker oder einem Molekül, das imstande ist, mit Avidin oder Streptavidin zu reagieren, markiert ist.

10. Verfahren nach einem der Ansprüche 1 bis 9, dadurch gekennzeichnet, daß die zu bestimmende Substanz die Substanz P oder Thyroxin ist.

11. Verfahren nach einem der Ansprüche 1 bis 9, dadurch gekennzeichnet, daß die zu bestimmende Substanz ein Hapten ist, das in der Gruppe enthaltend ACTH, Angiotensin, ANF, Bradykinin, Enkephalin, LHRH, Ocytocine, Vasopressin, den Neurokininen, Endothelin und Leukotrien $E_4$ gewählt wird.

FIG.1A

FIG.1B

FIG.1C

FIG.1D

FIG.1E

FIG. 2

FIG. 3

FIG. 4

FIG. 5

FIG. 6

FIG. 7

FIG. 8

FIG. 9

FIG. 10

FIG. 11